# EUROPÄISCHE PATENTSCHRIFT

(11) **EP 0 630 223 B1**
(45) Veröffentlichungstag und Bekanntmachung des Hinweises auf die Patenterteilung: **16.04.1997**
(21) Anmeldenummer: 93905289.0
(22) Anmeldetag: 03.03.1993
(51) Int. Cl.: A61H 23/04

(54) **VORRICHTUNG ZUR MECHANISCHEN BEEINFLUSSUNG DER LUNGE ZUR SCHLEIMLÖSUNG**
DEVICE FOR MECHANICALLY RELEASING MUCILAGE FROM THE LUNG
DISPOSITIF SERVANT A EXERCER UNE ACTION MECANIQUE SUR LES POUMONS POUR EN DECOLLER LE MUCUS

(30) Priorität: 06.03.1992 DE 4207054
(43) Veröffentlichungstag der Anmeldung: 28.12.1994
(73) Patentinhaber: MOLNAR, Zoltan, D-72461 Albstadt (DE)
(72) Erfinder: MOLNAR, Zoltan, D-72461 Albstadt (DE)
(74) Vertreter: Möbus, Rudolf, Dipl.-Ing.
(86) Internationale Anmeldenummer: EP9300481
(87) Internationale Veröffentlichungsnummer: WO9317650

(56) Entgegenhaltungen:
- WO-A-87/04067
- WO-A-89/12433
- DE-A- 3 238 425
- US-A- 3 483 862
- US-A- 4 977 889

## Beschreibung

Die Erfindung betrifft eine Vorrichtung zur mechanischen Beeinflussung der Lunge zur Schleimlösung gemaß dem Oberbegriff des Anspruches 1.

Zur Therapie von Lungenerkrankungen gehört das Abklopfen des Patienten im Lungenbereich, um eine Schleimlösung in der Lunge zu fördern. Dieses Abklopfen erfolgt bisher von Hand und erfordert geschultes Pflegepersonal, welches die richtige Handhaltung beim Abklopfen und die Richtungsfolge am Körper des Patienten erlernt hat. Bei der starken Überlastung des Pflegepersonals in Krankenhäusern und Heilstätten besteht die Gefahr, daß das Abklopfen nur noch sporadisch, zu rasch und nicht mehr mit der nötigen Sorgfalt durchgeführt wird. Außerdem macht sich hier auch das unterschiedliche Geschick des Pflegepersonals bemerkbar.

Aus der WO 87/04067 ist ein Massagebett bekannt, das mit einzeln ansteuerbaren Klopfkörpern ausgestattet sein kann und somit gegebenenfalls ebenfalls einen gewissen schleimlösenden Effekt auf die Lunge ausüben kann.

Der Erfindung liegt die Aufgabe zugrunde, eine Vorrichtung zu schaffen, mit welcher das Abklopfen eines Patienten auf effektive Weise durchgeführt werden kann, ohne daß hierzu Pflegepersonal erforderlich ist.

Die gestellte Aufgabe wird erfindungsgemäß durch eine Vorrichtung mit den Merkmalen des Anspruches 1 gelöst.

Die Vorrichtung ist so ausgebildet, daß sie von nicht bettlägrigen Patienten auch ohne Mithilfe von Pflegepersonal angewandt werden kann. Hierzu ist der Träger nach Art einer Weste mit zwei Vorderteilen und einem Rückenteil ausgebildet, und die Teile sind mindestens teilweise mittels längenverstellbarer Bänder miteinander gekoppelt und weisen jeweils mehrere Klopfkörper auf. Eine solche Weste läßt sich den unterschiedlichen Abmessungen der Oberkörper von Patienten anpassen. Das Ein- und Abschalten der Vorrichtung kann der Patient auch selbst vornehmen.

Bei der erfindungsgemäß ausgebildeten Vorrichtung läßt sich über eine Steuereinrichtung der Antrieb der einzelnen Klopfkörper oder von Klopfkörpergruppen nach einer gewünschten Folge steuern. Auch lassen sich der Abstand der Klopfkörper vom Patientenkörper und die Klopfstärke einstellen, wobei durch die Anordnung der Klopfkörper, durch ihre Ausbildung, ihre Folgesteuerung und eine gleichmäßige Klopfstärke eine optimale Behandlung sichergestellt ist, bei welcher auch keine Klopfstelle vergessen wird.

Die Vorrichtung läßt sich dabei so herstellen, daß sie den hygienischen Anforderungen, die an mit dem Körper in Berührung kommende Geräte gestellt sind, voll gerecht wird. Hierzu können die Klopfkörper vorteilhafterweise spritzwassergeschützt, aber auswechselbar zwischen zwei Außenwandungen des Trägers angeordnet sein, von denen mindestens die dem Patientenkörper zugewandte Innenwandung mindestens im Bereich der Klopfkörper flexibel gestaltet ist. Die geschlossenen Außenwandungen lassen sich leicht mit chemischen Mitteln desinfizieren, ohne daß dabei die Klopfkörper in Mitleidenschaft gezogen werden können, die ihrerseits durch die Außenwandungen auch einer direkten Berührung mit dem Patienten entzogen sind. Die Klopfkörper können als konkave Scheiben ausgebildet sein, deren hohle Seite dem Patientenkörper zugewandt ist und die auf ihrer dem Patientenkörper abgewandten Rückseite mit ihrer Antriebsvorrichtung gekoppelt sind. Zweckmäßig konnen die Klopfkörper als gummielastische Glockenkörper ausgebildet sein.

Die jedem Klopfkörper zugeordnete Antriebsvorrichtung kann unterschiedlicher Art sein, beispielsweise eine elektromagnetische, hydraulische oder pneumatische Antriebsvorrichtung. Aus Sicherheits- und auch aus Gewichtsgründen ist eine pneumatische Antriebsvorrichtung bevorzugt, zumal beim Einsatz der Vorrichtung in Krankenhäusern, wo Druckluftanschlüsse auch für andere Vorrichtungen zur Verfügung stehen. Eine pneumatische Antriebsvorrichtung für die Klopfkörper kann zweckmäßig mindestens eine über die Steuereinrichtung an eine Druckluftquelle anschließbare und entlüftbare Druckluftkammer mit veränderlichem Volumen aufweisen, wobei die Druckluftkammer beispielsweise eine unter dem Überdruck der Luft elastisch aufweitbare Wandung bildet oder aber durch einen starren Zylinder mit verstellbarem Kolben begrenzt sein kann.

Nachfolgend werden zwei Ausführungsbeispiele einer erfindungsgemäß ausgebildeten Vorrichtung anhand der beiliegenden Zeichnung näher erläutert.

Im einzelnen zeigen:
- Fig. 1a, 1b: die Vorderansicht und Rückansicht einer als Weste ausgebildeten Vorrichtung;
- Fig. 2: einen Querschnitt durch ein Westenteil entlang der Linie II - II in Fig. 1a;
- Fig. 3: eine stark vergrößerte Schnittdarstellung eines einzelnen Klopfkörpers;
- Fig. 4: eine Draufsicht auf eine als Matte ausgebildete Vorrichtung (nicht gemäß der vorliegenden Erfindung)
- Fig. 5: einen Querschnitt durch die Matte entlang der Linie V - V in Fig. 4;
- Fig. 6: eine schematisierte Darstellung der Antriebsvorrichtungen für die Klopfkörper der Matte und ihre gegenseitige Anordnung in einer pneumatischen Steuerschaltung.

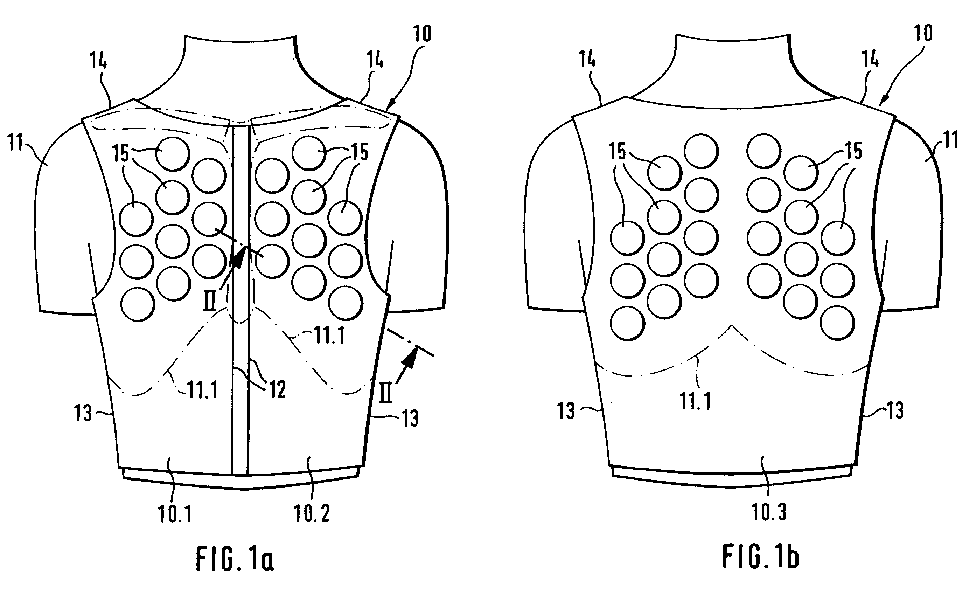

Die beiden Fig. 1a und 1b zeigen eine Vorrichtung zur mechanischen Beeinflussung der Lunge zur Schleimlösung, die als Weste 10 ausgebildet und einem Patienten 11 angelegt ist. Die Weste 10 besteht aus zwei Vorderteilen 10.1 und 10.2 sowie aus einem einstückigen Rückenteil 10.3. Die drei Westenteile 10.1 - 10.3 sind entlang ihrer vorderen Randbereiche 12, entlang ihrer seitlichen Randbereiche 13 und eventuell auch noch in ihren Schulterbereichen 14 mittels längenverstellbarer Bänder oder Schnüre miteinander verbunden, so daß sich die Weste 10 der unterschiedlichen Oberkörperweite der Patienten anpassen läßt. Aus Gründen der Übersichtlichkeit sind die Bänder und Schnüre in der Zeichnung nicht dargestellt. Solche Verbindungen von Bekleidungsstückteilen sind aber beispielsweise von Korsetts her bekannt. Es versteht sich, daß die beiden vorderen Westenteile 10.1 und 10.2 für männliche und weibliche Patienten einen gesonderten Zuschnitt und auch eine unterschiedliche Verteilung der in Fig. 1 durch Kreise angedeuteten einzelnen Klopfkörper 15 aufweisen können.

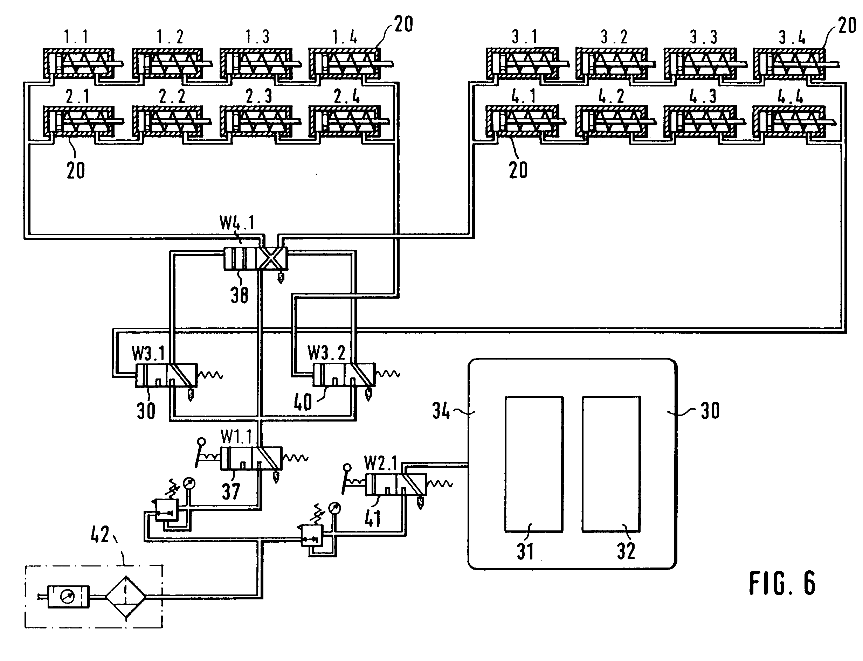

Jeder Klopfkörper 15 ist mit einer eigenen Antriebsvorrichtung versehen, die alle mit einer Steuervorrichtung in Verbindung stehen, die eine bestimmte Betätigungsfolge der Klopfkörper 15 bewirkt und die so ausgelegt sein kann, daß die in den beiden Vorderteilen 10.1 und 10.2 der Weste im Busenbereich von weiblichen Patienten befindlichen Klopfkörper 15 abschaltbar sind. Ein Beispiel für eine Steuereinrichtung ist in Fig. 6 gezeigt und wird nachfolgend noch beschrieben.

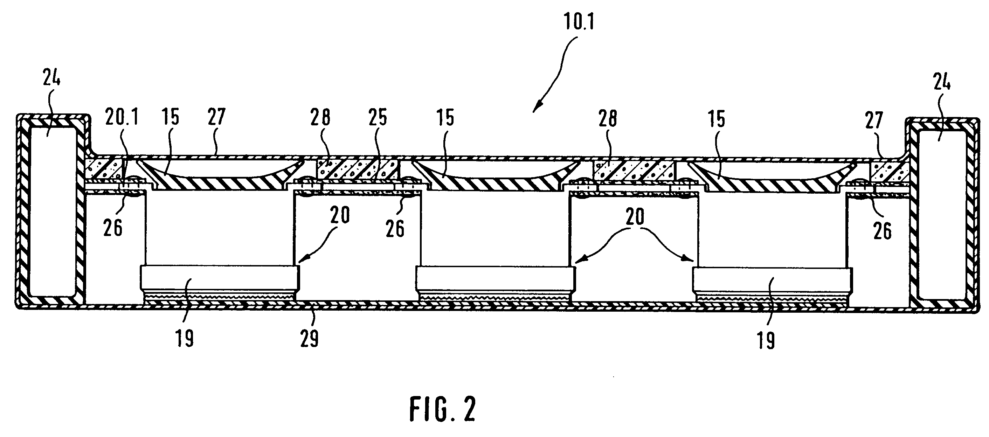

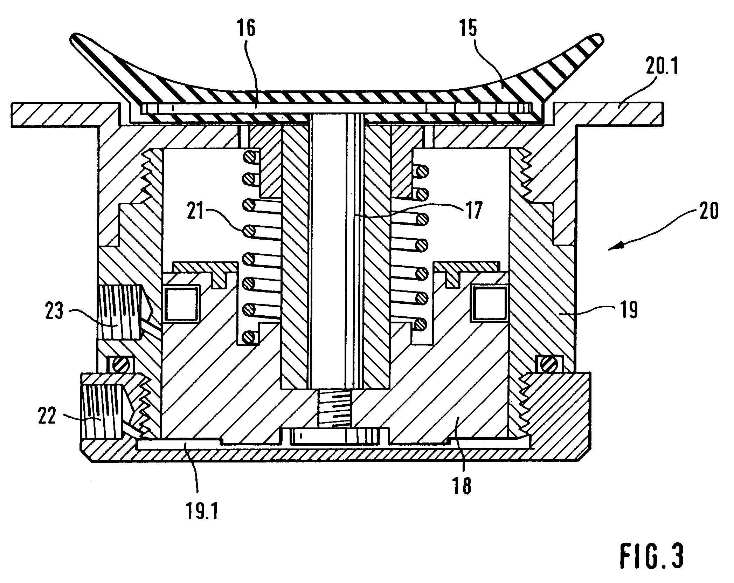

Bei der Weste 10 sind pneumatisch betätigte Klopfkörper vorgesehen. Die Klopfkörper 15 haben gemäß Fig. 2 die Form von gummielastischen flachen Glocken, die gemäß dem Schnittbild der Fig. 3 auf einer Abschlußscheibe 16 einer Kolbenstange 17 eines Kolbens 18 einer pneumatischen Antriebsvorrichtung 20 angeordnet sind, der in einem Zylindergehäuse 19 gegen die Kraft einer Rückstellfeder 21 mittels Druckluft verstellbar ist. Das Zylindergehäuse 19 weist für den Zylinderraum 19.1 zwei Anschlüsse 22 und 23 auf, von denen über die bereits erwähnte Steuereinrichtung der Anschluß 22 an eine Druckluftquelle und der Anschluß 23 mit dem Anschluß 22 der Antriebsvorrichtung 20 eines anderen Klopfkörpers 15 verbunden sein kann. Fig. 3 zeigt den Kolben 18 in seiner Ruhestellung. Beim Betrieb der Antriebsvorrichtung 20 bewegt sich der Kolben 18 in Fig. 3 nach oben und gibt nach einer gewissen Hubstrecke den Anschluß 23 frei.

Die pneumatische Antriebsvorrichtung 20 muß nicht als Zylinder/Kolben-Anordnung ausgebildet sein, sondern könnte auch als mit dem Klopfkörper 15 gekoppelter Pumpbalg ausgebildet sein, der über eine entsprechend angepaßte Steuereinrichtung in einer bestimmten Folge an eine Druckluftquelle angeschlossen und wieder entlüftet wird. Auch können die Klopfkörper 15 eine andere, von der Glockenform abweichende Form haben.

Der Zylinderkörper 19 der pneumatischen Antriebsvorrichtung 20 ist mit einem Montageflansch 20.1 versehen, über welchen die Antriebsvorrichtung 20 zusammen mit dem Klopfkörper 15 gemäß Fig. 2 in einem flexiblen Trägergewebe 25 der Westenteile 10.1 - 10.3 auswechselbar verankert ist. Zur Verankerung dienen bei dem dargestellten Ausführungsbeispiel Druckknöpfe 26. Der mit den Klopfkörpern 15 versehene Bereich der Westenteile 11.1 - 11.3 ist durch einen aufblasbaren Polsterkörper 24 begrenzt, der entlang des Westenteilrandes und der in Fig. 1a und 1b bezeichneten Rippenbögen 11.1 des Patienten 11 verläuft und mit welchem der Abstand der Klopfkörper 15 im Ruhezustand vom Patienten eingestellt werden kann, damit sich ein freier Klopfweg für die Klopfkörper 15 ergibt.

Über die glockenförmigen Klopfkörper 15 spannt sich eine flexible wasserdichte Folie 27, welche eine geschlossene Innenwandung der Westenteile bildet, die zwischen den einzelnen Klopfkörpern 15 über derartige Polstereinlagen 28 mit dem Trägergewebe 25 der Westenteile verbunden ist. Auch die Außenseite der Weste wird von einer geschlossenen wasserdichten, stabilen aber biegbaren Folie 29 gebildet, die gemäß Fig. 2 an den Stirnseiten der Zylindergehäuse 19 mittels Klettenverschlüssen verankert ist. Die Klopfkörper 15 mit ihren Antriebsvorrichtungen 20 sind also spritzwasserdicht zwischen der Außenfolie 29 und der Innenfolie 27 angeordnet, die aber zum Auswechseln von Klopfkörpern 15 abnehmbar oder durch Reißverschlüsse öffenbar sind.

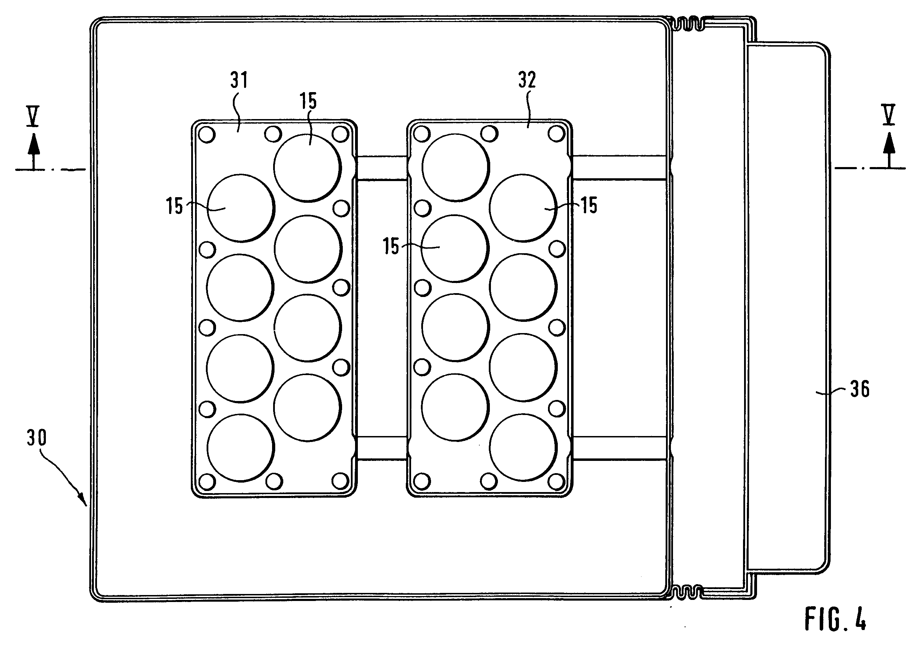

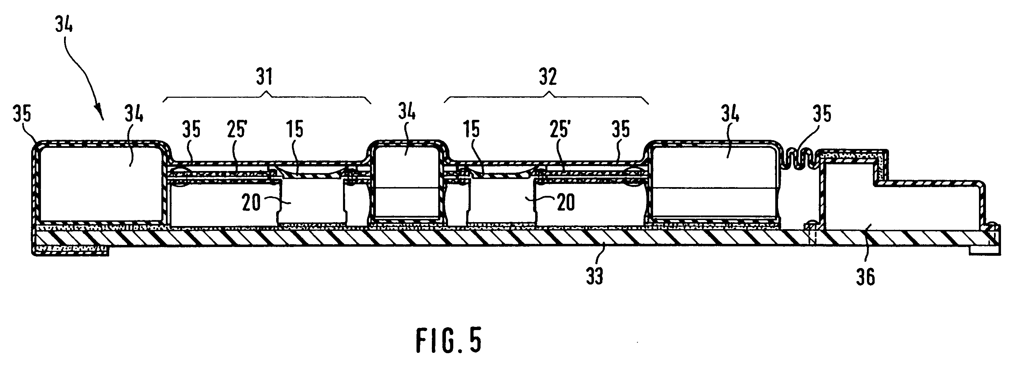

Fig. 4 zeigt eine Ausführungsform einer nicht unter der Definitionen der vorliegenden Erfindung fallenden Vorrichtung als Matte 30, in welcher zwei mit Klopfkörpern 15 versehene, gepolsterte Felder 31 und 32 ausgebildet sind. Wie das Schnittbild der Fig. 5 zeigt, besteht die Matte aus einer weitgehend starren Grundplatte 33, auf welche die beiden Felder 31 und 32 begrenzende und aufblasbare Polsterkammern 34 angeordnet sind. Mit den Polsterkammern 34, die als erstes aufgeblasen werden, läßt sich auch der Abstand der Klopfkörper in ihrer Ruhestellung vom Patientenkörper einstellen. Die Klopfkörper 15 haben den gleichen Aufbau wie beim Ausführungsbeispiel nach den Fig. 1 bis 3 und sind mit ihren pneumatischen Antriebsvorrichtungen 20 wieder in einem Trägergewebe 25' verankert. Beide Felder 31 und 32 sind zur Oberseite der Matte 30 hin mittels einer die gesamte Matte abdeckenden, durch Klettbänder gehaltenen, flexiblen Folie 35 abgedeckt und nach unten durch die Grundplatte 33 verschlossen. Auf einer Seite weist die Matte 30 ein ebenfalls auf der Grundplatte 33 angeordnetes Steuerbord 36 auf, das ebenfalls teilweise durch die flexible Folie 35 abgedeckt ist und in welchem sich die Organe der Steuereinrichtung für den pneumatischen Antrieb der Klopfkörper 15 befinden und Bedienungstasten angeordnet sind. In der Zeichnung sind die Steuerteile, also Pneumatikventile, und Bedienungstasten nicht dargestellt. Das Bedienungsbord 36 ragt seitlich unter einem auf der Matte 30 liegenden Patienten hervor, so daß die Bedienungstasten auch dem Patienten zugänglich sind.

Fig. 6 zeigt in schematischer Anordnung die in den beiden Feldern 31 und 32 der Matte 30 verteilten pneumatischen Antriebsvorrichtungen 20 und in der Steuereinrichtung verwendete Steuer- und Schaltventile 37 - 41. Von einer schematisch angedeuteten Druckluftquelle 42 aus werden über ein Schaltventil 37 und ein Verteilerventil 38 die in zwei Gruppen hintereinandergeschalteten pneumatischen Antriebsvorrichtungen 20 betätigt. Über ein steuerbares Schaltventil 41 wird in gewünschtem Maße Druckluft in die aufblasbaren Polsterkammern 34 geleitet. Anstelle aufblasbarer Polsterkammern 34 könnten auf die Grundplatte 33 auch elastisch verformbare Polsterkörper 34 aufgesetzt sein.

Wie bereits erwähnt, können die Antriebsvorrichtungen 20 und kann die Steuereinrichtung unterschiedlich ausgebildet sein, weshalb auf eine genaue Beschreibung der in Fig. 6 dargestellten Steuereinrichtung verzichtet wird. Anstelle pneumatischer Antriebsvorrichtungen 20 können unter erhöhtem Sicherheitsaufwand im Hinblick auf die elektrische Isolation auch elektromagnetische Vorrichtungen Verwendung finden, die den Anschluß der Vorrichtung an das elektrische Versorgungsnetz erlaubt und damit deren Anwendungsbereich erweitert.

## Patentansprüche

1. Vorrichtung zur mechanischen Beeinflussung der Lunge zur Schleimlösung mit einem Träger (10; 30), der mit mehreren einzeln oder gruppenweise antreibbaren Klopfkörpern (15) besetzt ist, dadurch gekennzeichnet, daß der Träger nach Art einer Weste (10) mit zwei Vorderteilen (10.1, 10.2) und einem Rückenteil (10.3) ausgebildet ist, deren Teile mindestens teilweise mittels längenverstellbarer Bänder miteinander gekoppelt sind und jeweils mehrere Klopfkörper (15) aufweisen.

2. Vorrichtung nach Anspruch 1, dadurch gekennzeichnet, daß sie eine Steuereinrichtung für eine Folgesteuerung der Antriebsvorrichtungen (20) der einzelnen Klopfkörper (15) oder von Klopfkörpergruppen aufweist.

3. Vorrichtung nach einem der Ansprüche 1 bis 2, dadurch gekennzeichnet, daß die Klopfkörper (15) wasserdicht zwischen zwei Außenwandungen (27, 29; 33, 35) des Trägers (10; 30) angeordnet sind, von denen mindestens die dem Patientenkörper zugekehrte Innenwandung (27; 35) mindestens im Bereich der Klopfkörper (15) flexibel gestaltet ist.

4. Vorrichtung nach einem der Ansprüche 1 bis 3, dadurch gekennzeichnet, daß die Klopfkörper (15) als dem Patientenkörper zu konkave Scheiben ausgebildet sind, die auf ihrer dem Patientenkörper abgewandten Rückseite mit ihrer Antriebsvorrichtung (20) gekoppelt sind.

5. Vorrichtung nach Anspruch 4, dadurch gekennzeichnet, daß die Klopfkörper (15) als gummielastische Glockenkörper ausgebildet sind.

6. Vorrichtung nach einem der Ansprüche 1 bis 5, dadurch gekennzeichnet, daß die Klopfkörper (15) aufweisenden Bereiche des Trägers (10; 30) durch Polsterkammern (24; 34) begrenzt sind, die mindestens teilweise zur Einstellung des Abstandes der im Ruhezustand befindlichen Klopfkörper (15) vom Körper des Patienten (11) aufblasbar sind.

7. Vorrichtung nach einem der Ansprüche 1 bis 6, dadurch gekennzeichnet, daß die Klopfkörper (15) mit pneumatischen Antriebsvorrichtungen (20) versehen sind, die mindestens eine über die Steuereinrichtung an eine Druckluftquelle (42) anschließbare und entlüftbare Druckluftkammer (19.1) mit veränderlichem Volumen aufweisen.

8. Vorrichtung nach einem der Ansprüche 1 bis 6, dadurch gekennzeichnet, daß die Klopfkörper (15) mit elektromagnetischen und elektrisch folgegesteuerten Antriebsvorrichtungen gekoppelt sind.

## Claims

1. A device for mechanically influencing the lungs for the release of mucus, having a support (10; 30) which is covered by a plurality of striker bodies (15) which can be driven individually or in groups, characterised in that the support is constructed in the manner of a waistcoat (10) with two front parts (10.1, 10.2) and a back part (10.3), the parts of which are at least partly coupled to each other by means of longitudinally adjustable tapes, and each comprise a plurality of striker bodies (15).

2. A device according to claim 1, characterised in that it comprises a controller for the sequential control of the drive devices (20) of the individual striker bodies (15) or of groups of striker bodies.

3. A device according to either one of claims 1 to 2, characterised in that the striker bodies (15) are disposed in a water-tight manner between two external walls (27, 29; 33, 35) of the support (10; 30), of which at least the inner wall (27; 35) facing the patient's body is of flexible construction, at least in the region of the striker bodies (15).

4. A device according to any one of claims 1 to 3, characterised in that the striker bodies (15) are constructed as discs which are concave towards the patient's body and which are coupled to their drive device (20) on their back face remote from the patient's body.

5. A device according to claim 4, characterised in that the striker bodies (15) are constructed as cup bodies of rubber-like resilience.

6. A device according to any one of claims 1 to 5, characterised in that the regions of the support (10; 30) which comprise the striker bodies (15) are delimited by cushioning chambers (24; 34) which are at least partially inflatable in order to adjust the distance of the striker bodies (15) in their inoperative state from the body of the patient (11).

7. A device according to any one of claims 1 to 6, characterised in that the striker bodies (15) are provided with pneumatic drive devices (20) which comprise at least one compressed air chamber (19.1) of variable volume, which can be connected via the controller to a compressed air source (42) and which can be deaerated.

8. A device according to any one of claims 1 to 6, characterised in that the striker bodies (15) are coupled to electromagnetic drive devices which are sequentially controlled electrically.

## Revendications

1. Dispositif servant à exercer une action mécanique sur les poumons pour en décoller le mucus, avec un support (10 ; 30) qui est garni de plusieurs corps percutants (15) pouvant être actionnés individuellement ou en groupe, caractérisé en ce que le support est conçu à la manière d'un gilet (10) avec deux parties de devant (10.1, 10.2) et une partie de dos (10.3), lesdites parties étant reliées entre elles au moins partiellement à l'aide de bandes réglables en longueur et comportant chacune plusieurs corps percutants (15).

2. Dispositif selon la revendication 1, caractérisé en ce qu'il comporte un dispositif de commande pour commander séquentiellement les dispositifs d'actionnement (20) des différents corps percutants (15) ou de groupes de corps percutants.

3. Dispositif selon l'une des revendications 1 à 2, caractérisé en ce que les corps percutants (15) sont disposés de manière étanche à l'eau entre deux parois extérieures (27, 29 ; 33, 35) du support (10 ; 30), dont au moins la paroi intérieure (27 ; 35) tournée vers le corps du patient est conçue de manière souple au moins dans la zone des corps percutants (15).

4. Dispositif selon l'une des revendications 1 à 3, caractérisé en ce que les corps percutants (15) sont conçus sous la forme de disques concaves par rapport au corps du patient, qui, sur leur face arrière opposée au corps du patient, sont reliés à leur dispositif d'actionnement (20).

5. Dispositif selon la revendication 4, caractérisé en ce que les corps percutants (15) sont conçus sous la forme de corps formant cloches possédant l'élasticité du caoutchouc.

6. Dispositif selon l'une des revendications 1 à 5, caractérisé en ce que les zones du support (10 ; 30) comportant des corps percutants (15) sont délimitées par des chambres de rembourrage (24 ; 34) qui peuvent être gonflées au moins en partie pour régler l'écartement des corps percutants (15) se trouvant en position de repos par rapport au corps du patient (11).

7. Dispositif selon l'une des revendications 1 à 6, caractérisé en ce que les corps percutants (15) sont munis de dispositifs d'actionnement pneumatiques (20) qui comportent au moins une chambre à air comprimé (19.1) à volume variable pouvant être reliée à une source d'air comprimé (42) par l'intermédiaire du dispositif de commande et pouvant être vidée de son air.

8. Dispositif selon l'une des revendications 1 à 6, caractérisé en ce que les corps percutants (15) sont couplés à des dispositifs d'actionnement électromagnétiques et commandés électriquement de manière séquentielle.
